# EUROPEAN PATENT SPECIFICATION

(11) **EP 2 164 507 B1**
(45) Date of publication and mention of the grant of the patent: **11.07.2012**
(21) Application number: 08825917.1
(22) Date of filing: 13.05.2008
(51) Int. Cl.: A61K 38/05, A61P 11/06, A61P 37/08

(54) **TREATMENT OF ALLERGIC DISEASE WITH IMMUNOMODULATOR COMPOUNDS**
BEHANDLUNG ALLERGISCHER ERKRANKUNGEN MIT IMMUNMODULATOR-VERBINDUNGEN
TRAITEMENT D'UNE MALADIE ALLERGIQUE AVEC DES COMPOSÉS IMMUNOMODULATEURS

(30) Priority: 17.05.2007 RU 2007118237; 22.08.2007 US 957201 P
(43) Date of publication of application: 24.03.2010
(73) Proprietor: Sciclone Pharmaceuticals, Inc., Foster City, California 94404 (US)
(72) Inventor: KOLOBOV, Alexandr A., 188640 Sestroretsk (RU); SIMBIRTSEV, Andrey S., 194021 St. Petersburg (RU)
(74) Representative: Vierheilig, Achim
(86) International application number: PCT/US2008/006072
(87) International publication number: WO 2008/143824

(56) References cited:
- US-A- 5 916 878
- US-A1- 2002 177 226
- US-A1- 2007 087 974
- "The Penguin Dictionary of Chemistry, Second Edition", 1990, PENGUIN GROUP, ENGLAND pages 224-225,

## Description

### BACKGROUND OF THE INVENTION

### Field of the Invention

The present invention relates to the field of treatment of allergic disease.

### Description of Background Art

Atopic bronchial asthma is a widespread disease in industrially developed countries. The role of T lymphocytes and the cytokines produced by them is now beyond question in the pathogenesis of asthma. According to one of the leading hypotheses, a shift in the balance of T helpers in the direction of T helpers of the second type, accompanied by predominant production of interleukin-4 (IL-4) is an important factor in triggering and maintaining the asthmatic process in lung tissue.

Allergic states, in conjunction with a deterioration in immune status of the body of the patient and the progressive contamination of the environment, are widespread in industrially developed countries.

Preparations that belong to different pharmaceutical groups are now used to prevent and treat allergies. In particular, the use of anti-inflammatories is proposed (aspirin, ibuprofen, voltaren, hydrocortisone); antihistamine preparations (dimedrol, pipolphen) are used in different schemes and combinations with calcium channel blockers of fat cells (sodium chromalin) and hormonal therapy, combined with symptomatic agents, bronchodilators, adsorbents and various homeopathic preparations of natural and synthetic origin, corticosteroids, etc. (RU 2240126, 2002; RU 2139100, 1996; RU 21167691, 1994; Handbook for the Practical Physician, Moscow, Meditsina, 1988, Vol.1, pages 60-68; SU 1544438,1990; Immunocorrection on Pulmonology/edited by A. G. Chuchalin, Moscow, Meditsina, 1989, pages 202-210; Allergology. General Allergology, Vol. 1/edited by G. B. Fedoseev, St. Petersburg, Nordmed Publishers, 2001, 816 pages).

A shortcoming of most known preparations is the relatively low efficiency, and also the large number of contraindications. In particular, sodium cromoglycate (intal) is only effective during Inhalation exposure and only in the case of slight manifestations of allergic disease; antihistamine preparations are ineffective in asthma, and the aspirin triad exhibits pronounced side effects (RU 2170091, 1994), etc.

In recent years, a theory has been considered generally accepted, according to which allergic diseases are caused by disorders in regulation on the immune system associated with activation of allergen-specific clones of helper T-lymphocytes, called Type 2 T-helper cells (Th2) (Allergology. General Allergology, Vol. 1/edited by G. B. Fedoseev, St. Petersburg, Nordmed Publishers, 2001, 816 pages). In this connection, the use of mutual regulation of T-helper clones by introducing differentiation inductors of the opposing Type 1 T-helper clones (Th1) or using cytokines synthesized by them has been proposed for treatment of allergy. In particular, interteukin-12, gamma interferon (IFN- y) and other substances have been used for these purposes.

However, the obtained results proved to be insufficiently reliable and, In many cases, contradictory. Thus, it was demonstrated that the level of IFN-γ correlates with the intensity of the inflammatory allergic reaction and the severity of the clinical manifestations of the disease (Leonardi A., Cumow S., Zhan H., Calder V. Multiple cytokines in human tear specimens in seasonal and chronic allergic eye disease and in conjunctival fibroblast culture. Clin. Exp. Allergy, 2006, V. 36, p. 777-784). At the same time, in a number of asthma models in mice, it was demonstrated that administration of IFN-γ in animals can lead to an increase in hyperreactivity of the bronchi (Hessel E., Van Oosterhout A., Van Ark I. et al. Development of airway hyperresponsiveness Is dependent of IFN-amma and independent of eosinophil infiltration. Am. J. Respir. Cell. Mol. Biol., 1997, V. 16, p. 325-335).

Administration of a preparation of recombinant interleukin-12 in patients with asthma caused a reduction in eosinophilia, but did not lead to a reduction In hyperreactivity and symptoms of bronchospasm on a background of suffieciently high general toxicity (Bryan S., O'Connor B., Matti S. et al. Effects of recombinant human IL-12 on eosinophils, airway hyper-responsiveness, and the late asthmatic response. Lancet, 2000, V. 356, p. 2149-2153).

Interleukin-18 (IL-18) has been used for treatment of allergic disease (Wild J., Sigounas A., Sur N. et al. IFN-γ-inducing factor (IL-18) increases allergic sensitization, serum IgE, Th2 cytokines, and airway eosinophilia in a mouse model of allergic asthma. J. Immunol., 2000, V. 164, p. 2701-2710). Administration of IL-18 in animals, which overall exerts a positive effect on the organism, in some case, leads to an increase in allergic manifestations. Apparently, this is associated with the fact that IL-18 is capable of activating both types of T-helper clones and intensifying synthesis of both IFN-γ and IL-4.

A shortcoming of IL-18 and other preparations mentioned above is the polyclonal activation of the immune system, the absence of selectivity of the effect, and the high toxicity, which was found during clinical trials.

US 2002/0177226 A1 teaches the treatment of autoimmune diseases using sterioisomeric forms of Glu-Trp dipeptides. Sterioisomeric forms are D- and L- isomers and do not include different types of peptide bonds. Peptides are generally and naturally synthesized with a peptide bonds, and the reference does not suggest a different type of peptide bond.

There remains a need in the art for compounds suitable for treating, preventing, inhibiting or reducing allergic disease or its effects in a subject.

### SUMMARY OF THE INVENTION

Subject matter of the present invention is an immunomodulator compound for use in treating, preventing, inhibiting or reducing allergic disease or its effects in a subject, as well as the use of the immunomodulator compound for the preparation of a medicament for treating, preventing, inhibiting or reducing allergic disease or its effects in a subject, as claimed in the independent claims. Embodiments of the invention are claimed in the dependent claims.

The immunomodulator compound is for use in a method of treatment for treating, preventing, inhibiting or reducing allergic disease or its effects in a subject, the method comprising administering to the subject an effective amount ot the immunomodulator compound of formula A:

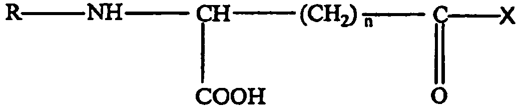

In Formula A, n is 2, R is hydrogen, acyl, alkyl or a peptide fragment, and X is L-tryptophan or D-tryptophan.

### BRIEF DESCRIPTION OF THE DRAWINGS

Fig. 1 graphically depicts results with one embodiment.

Fig. 2 graphically depicts further results with the Invention.

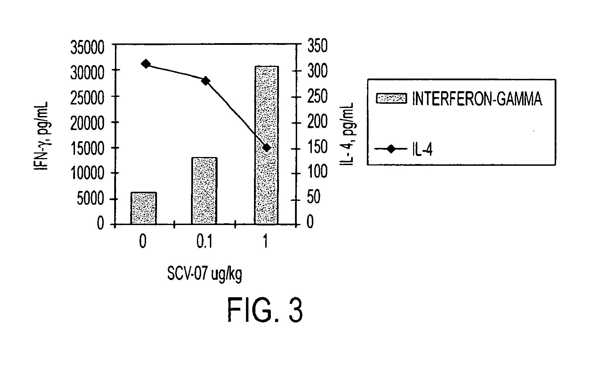

Fig. 3 graphically depicts further results with the invention.

Fig. 4 graphically depicts further results with the invention.

Fig. 5 graphically depicts further results with the Invention.

Fig. 6 graphically depicts further results with the invention.

Fig. 7 graphically depicts further results with the invention.

Fig. 8 graphically depicts further results with the invention.

### DESCRIPTION OF THE PREFERRED EMBODIMENTS

In accordance with one embodiment, the present invention relates to an immunomodulator compound for use in a method of treatment for treating, preventing, inhibiting, or reducing an allergic disease such as asthma or its effects in a subject, preferably a human patient. In certain embodiments, atopic bronchial asthma is treated.

Immunomodulator compounds in accordance with the present invention comprise immunomodulators of Formula A:

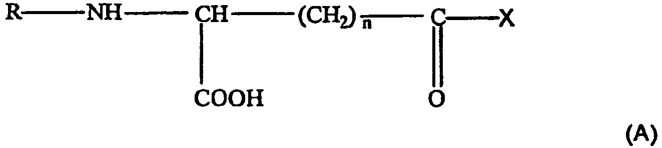

In Formula A, n is 2, R is hydrogen, acyl, alkyl or a peptide fragment, and X is L-tryptophan or D-tryptophan.

Appropriate acyl or alkyl moieties for "R" are: branched or unbranched alkyl groups of 1 to about 6 carbons, acyl groups from 2 to about 10 carbon atoms, and blocking groups such as carbobenzyloxy and t-butyloxycarbonyl. Preferably the carbon of the CH group shown in Formula A has a stereoconfiguration, that is different from the stereoconfiguration of X.

Preferred embodiments utilize compounds such as γ-D-glutamyl-L-tryptophan, γ-L-glutamyl-L-tryptophan, γ-L-glutamyl-Nᵢₙ-formyl-L-tryptophan, N-methyl-γ-L-glutamyl-L-tryptophan, N-acetyl-γ-L-glutamyl-L-tryptophan, and γ-L-glutamyl-D-tryptophan. Particularly preferred embodiments utilize γ-D-glutamyl-L-tryptophan, sometimes referred to as SCV-07. These compounds, methods for preparing these compounds, pharmaceutically acceptable salts of these compounds and pharmaceutical formulations thereof are disclosed in U.S. Patent No. 5,916,878.

SCV-07, γ-D-glutamyl-L-tryptophan, is a member of a class of immunomodulatory drugs that possess γ-glutamyl or β-aspartyl moieties, which was discovered by Russian scientists and is being examined for efficacy in several indications In the U.S. by SciClone Pharmaceuticals, Inc. SCV-07 possesses a number of immunomodulatory activities in vivo and in vitro. SCV-07 increases Con-A-induced thymocyte and lymphocyte proliferation, increases Con-A-induced interieukin-2 (IL-2) production and IL-2 receptor expression by spleen lymphocytes, and stimulates expression of Thy-1.2 on bone marrow cells. In vivo, SCV-07 has a strong immunostimulatory effect on 5-FU-immune-suppressed animals and in a model of immunization with sheep red blood cells.

The Formula A compounds may be administered at any effective dosage, e.g., at dosages in the range of about 0.001-10 mg. Dosages may be administered one or more times per week, e.g., on a daily basis, with dosages administered one or more times per day. Administration can be by any suitable method, including orally, nasally, transdermally, sublingually, by injection, periodic infusion, continuous infusion, and the like. The dosages may be administered by intramuscular injection, although other forms of injection and infusion may be utilized, and other forms of administration such as oral or nasal inhalation or oral ingestion may be employed.

In preferred embodiments, the compounds of Formula A are administered at a dosage within a range of about 0.001-10 mg, more preferably 0.01-1 mg, most preferably at a dosage of about 0.1 mg.

Dosages may also be measured in micrograms per kilogram, with dosages in the range of about 0.00001-100 mg/kg, more preferably within the range of about 0.0001-1 mg/kg, still more preferably about 0.001-0.01 mg/kg.

SCV-07 contains the active principle dipeptide-γ-D-Glu-L-Trp (gamma-D-glutamyl-L-tryptophan), obtained by chemical synthesis and purified to homogeneity by high-performance liquid chromatography (RU 2091389, 1997; RU 2120298, 1998). The preparation was previously used to restore immunity in infectious diseases and after surgical operations. The preparation has immune-stimulating effects at dosages as low as from 0.00001 mg to 0.01 mg per kg of body weight, i.e., it exerts its effect in extremely low concentrations.

To treat allergic diseases, the preparation is administered at dosages permitted by the pharmacopeia. Depending on the form of administration to the body and the characteristics of the disease, in certain embodiments the daily dose of the preparation is from 0.1 to 1.5 mg. Use of the preparation in lower doses is possible, but reduces the therapeutic effect, and its use in a dose greater than 1.5 mg is not economically expedient. The preparation is non-toxic in doses that exceed the employed ones by a minimum of 100,000 times.

When used parenterally (intramuscular or intra-abdominal), in certain embodiments the preparation is used in a volume of 1 mL isotonic sodium chloride solution, in which the active principle is dissolved (γ-D-Glu-L-Trp 100 µg) with excipients (D-mannitol 9.0 mg, sodium chloride 1.0 mg). The single dose in certain embodiments is 0.1 mg (0.001 mg/kg). Treatment with the preparation is conducted in certain embodiments in the form of a course of 5-10 injections.

When used perorally, the preparation is used in certain embodiments in the form of tablets, containing 0.35 or 1.5 mg active principle. The preparation is used in certain embodiments once or twice a day for 1-2 weeks.

Included are biologically active analogs having substituted, deleted, elongated, replaced, or otherwise modified portions which possess bioactivity substantially similar to that of SCV-07, e.g., an SCV-07 derived peptide having sufficient homology with SVC-07 such that it functions in substantially the same way with substantially the same activity as SCV-07.

According to one embodiment, a Formula A compound may be administered to a patient so as to substantially continuously maintain an effective amount of the Formula A compound in the patient's circulatory system during a treatment or prevention period. Although much longer treatment periods are contemplated in accordance with the present invention, embodiments of the invention include substantially continuously maintaining an effective amount of the Formula A compound in the patient's circulatory system during treatment periods of at least about 6, 10, 12 hours, or longer. In other embodiments, treatment periods are for at least about a day, and even for a plurality of days, e.g., a week or longer. However, it is contemplated that treatments, as defined above, in which effective amounts of the Formula A compound are substantially continuously maintained in the patient's circulatory system, may be separated by non-treatment periods of similar or different durations.

In accordance with one embodiment, the Formula A compound is continuously infused into a patient, e.g., by intravenous infusion, during the treatment period, so as to substantially continuously maintain an effective amount of the Formula A compound in the patient's circulatory system. The infusion may be carried out by any suitable means, such as by minipump.

Alternatively, an injection regimen of the Formula A compound can be maintained so as to substantially continuously maintain an effective amount of the Formula A compound in the patient's circulatory system. Suitable injection regimens may include an injection every 1, 2, 4, 6, etc. hours, so as to substantially continuously maintain the effective amount of the Immunomodulator compound peptide in the patient's circulatory system during the treatment period.

Although it is contemplated that during continuous infusion of the Formula A compound, administration will be for a substantially longer duration, according to one embodiment the continuous infusion of the Formula A compound is for a treatment period of at least about 1 hour. More preferably, continuous infusion is carried out for longer periods, such as for periods of at least about 6, 8, 10, 12 hours, or longer. In other embodiments, continuous infusion is for at least about one day, and even for a plurality of days such as for one week or more.

In some embodiments, the Formula A compound is present in a pharmaceutically acceptable liquid carrier, such as water for injection, saline in physiological concentrations, or similar.

Effective amounts of Formula A compound can be determined by routine dose-titration experiments.

The Formula A compound also can be administered with other asthma-treating agents.

The following non-limiting examples illustrate the invention.

### Example 1: Treatment of Atopic Brochial Asthma

SCV-07 is a synthetic immunomodulator having a broad spectrum of action on the immune system and, in particular, capable of intensifying IL-2 and interferon-γ production. This activity apparently stems from a shift in balance of T helpers under the influence of the preparation toward T helpers of the first type. The objective of this work was to investigate the effect of SCV-07 on experimental allergic asthma in mice. For this purpose we used a model of allergic ovalbumin asthma with inhalation administration of the allergen. SCV-07 was administered to the animals after preliminary systemic immunization for modeling the situation of encounter of a presensitized organism with the allergen.

### MATERIALS AND METHODS

### Modeling of experimental allergic asthma

Female, non-pathogenic mice of the Balb/C line ("Pushchtino" laboratory animal breeders) were kept under conditions of an SPF-vivarium at a constant temperature, 12-hour day/night cycle, and received sterile feed and water ad libitum. In two series of experiments, mice from 6-8 weeks and 18-20 weeks of age were used at the beginning of the experiment, respectively.

The animals were immunized twice at 5-day intervals with an ovalbumin solution (Sigma) in an adjuvant based on ammonium sulfate at a dosage of 8 µg per mouse. SCV-07 was administered intra-abdominally in a volume of 200 µL at a dosage of 0.1 or 1.0 µg/kg in PBS once a day from 6 through 11 days after the first immunization. The control group received physiological saline on the same days. Each group comprised 6 to 10 animals. On the 12^{th}, 16^{th} and 20^{th} days after the first immunization, the animals were then placed in a hermetic box, in which an aerosol of 1% ovalbumin solution (OvA) in PBS was generated with an ultrasonic nebulizer LD-207U (Little Doctor) for 1 minute with continuous mixing of air. The duration of exposure of the animals to the aerosol was 15 minutes. Analysis of the severity of experimental allergic asthma was carried out 24 hours after the last aerosol exposure.

Analysis of the changes in severity of experimental allergic asthma during use of SCV-07 was carried out according to the cytological composition of bronchoalveolar lavage, histologic investigations, and the determination of the titers of antigen-specific IgE antibodies.

The mice were killed by intra-abdominal administration of a lethal dose of sodium barbital. Bronchoalveolar lavage (BAL) was obtained by washing the lungs through the trachea with 1 mL PBS solution. The BAL cells were precipitated by centrifuging, the precipitate was resuspended, the cell concentration was counted in a Goryaev chamber, and smears were prepared. The smears were dried, fixed and stained with Romanovskii dye. The cytological composition of the bronchoalveolar lavage was investigated under a light microscope with oil immersion (eyepiece × 16, objective × 100). The numbers of monocyte/macrophages, neutrophils, eosinophils and lymphocytes per 200 cells were counted, in which no less than 5 visual fields were viewed. The relative number of cells of each cell population was expressed as the percentage of the total number of cells.

After taking the bronchoalveolar lavage, the lungs were fixed in 4% paraformaldehyde for 3-7 days, passed through a Peterfi solution according to increasing alcohols (2% celluidine-castor oil) and chloroform, and poured into paraffin. Sections of 5 µm thick were prepared. The sections were stained with hematoxylin-eosin to evaluate the intensity of cell infiltration and Schiff-iodic acid to count the number of goblet cells.

Quantitative evaluation of the peribronchial and perivascular infiltration was accomplished morphometrically with the Scion Image program package. Three bronchi and three vessels were analyzed on sections of the lungs from each animal. The area of the infiltration was calculated as the difference between the area of the tissue containing bronchi or vessels and the infiltration surrounding it, and the area of the vessel or bronchus itself. The indices of infiltration were calculated as the ratio of the area of the infiltration to the area of the vessel or bronchus. Experimental groups were comparable in terms of area of bronchi and vessels included in analysis (the differences between groups were not significant, p > 0.6).

The number of goblet and epithelial cells on the sections of the bronchi were calculated in no less than five visual fields on sections from the lungs from each animal and expressed as the percentage of the total number of cells. The experimental groups were comparable in dimensions of the bronchi included in analysis (the differences between groups, in terms of number of cells in the bronchus, were not significant, p > 0.5).

The peripheral blood was collected from the retro-orbital sinus. The titer of anti - OvA IgE in the serum and bronchoalveolar lavage was determined with solid phase IFA according to the following scheme. Rat antibodies to murine IgE labeled with biotin (Caltag) were introduced to a 96-well plate for IFA ((Coming Costar) in the dilution recommended by the manufacturer (100 µL/well), incubated for an hour at room temperature and washed. Serum samples or BAL were then introduced in successive dilutions and incubated at room temperature for an hour and washed. OvA was then added, labeled with horseradish peroxidase, incubated on a shaker for an hour at room temperature, washed and the substrate for peroxidase introduced (OPD, Sigma) and incubated for 15 minutes. The reaction was stopped by adding 1 M H₂SO₄ and the plates were analyzed on a Victor2 spectrophotometer (Wallac) at a wavelength of 450 nm. For quantitative comparison of the titers of the antigen-specific IgE after subtracting the background, we calculated the average optical density in each dilution for samples of the animal control group, and expressed the optical densities in the samples from all animals in the experiment in corresponding dilution as the percentages of the values obtained.

The statistical processing of the results was carried out with the Microsoft Excel and SPSS programs for Windows v.13. The normality of the distribution was determined with the Kholmogorov-Smimov criterion. To evaluate the significance of the differences between groups for normally distributed values, we used the two-sided Student test with unequal deviations, for non-normally distributed differences - the Mann-Whitney test. The differences were considered significant at p < 0.05. Unless otherwise stated, the data are presented in the form of means ± standard deviation.

According to the obtained results, analysis of the cell composition of BAL in the experiments on young animals (age 6-8 weeks at the beginning of the experiment) did not show a significant effect of SCV-07. The total cellularity of the BAL in all groups was roughly the same (2.00 ± 0.34 million cells in the control group, 1.96 ± 0.99 million in the group that received 0.1 µg/kg of the preparation, and 2.21 ± 1.16 million in the group that received 1 µg/kg). By comparing the relative percentages of individual populations of leukocytes in BAL, we noted a significantly lower percentage of eosinophils and a higher percentage of macrophages in the group that received 1 µg/kg SCV-07, in comparison with the control animals (see Table 1). The absolute amounts of cells of the individual populations in BAL did not differ significantly between groups.

**Table 1. Percentage composition of BAL cells (animals 6 to 8 weeks).**

| | Groups of animals | | |
|---|---|---|---|
| Index | Control | SCV-07 0.1 µg/kg | SCV-07 1 µg/kg |
| Eosinophils, % | 65.1±5.1 | 57.4±21.3 | 56.7±9.5* |
| Monocytes/Macrophages , % | 32.9±5.3 | 41.2±20.7 | 40.6±9.4* |
| Neutrophils, % | 1.2±1.0 | 0.9±0.8 | 2.1±0.9 |
| Lymphocytes, % | 0.8±0.8 | 0.4±0.5 | 0.6±0.7 |

| | | | |
|---|---|---|---|
| * p < 0.05 in comparison with the control group | | | |

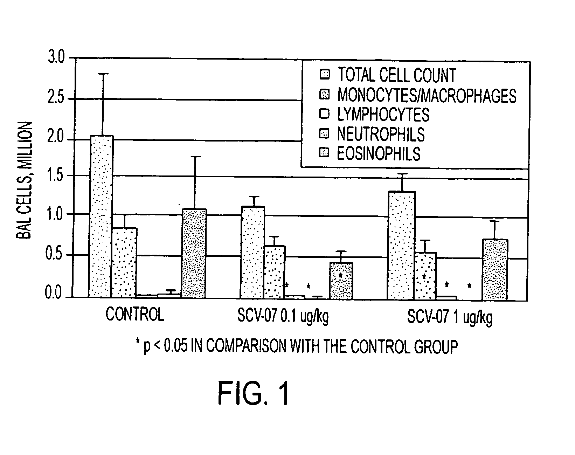

In experiments on animals from 18 to 20 weeks of age the changes in cellular composition of BAL and the groups that receive SCV-07 were essentially different. We noted a significant reduction in the total number of cells in the lavages in the group that received 0.1 µg/kg of the preparation (1.12±0.15 million in comparison with 2.03±0.78 million in the control group, p < 0.05), and a distinct tendency toward a reduction in this index in the group that received 1 µg/kg SCV-07 (1.32±0.23 million). In the absence of significant differences in the percentage content of leukocytes of the individual subpopulations, analysis of the absolute values showed a significant reduction in the number of eosinophils, neutrophils and lymphocytes in the group that received 0.1 µg/kg SCV-07, and monocytes/macrophages, neutrophils and lymphocytes in the group that received 1.0 µg/kg of the preparation (see Table 2, Figure 1, wherein SCV-07 is referred to as SCV-07).

**Table 2. Subpopulation composition of BAL cells (animals 18 to 20 weeks).**

| | Groups of animals | | |
|---|---|---|---|
| Index | Control | SCV-07 0.1 µg/kg | SCV-07 1 µg/kg |
| Eosinophils, thousand | 1151±655 | 456±144* | 690±247 |
| Monocytes/Macrophages , thousand | 806±178 | 629±134 | 586±144* |
| Neutrophils, thousand | 44±33 | 17±14* | 18±10* |
| Lymphocytes, thousand | 30±9 | 22±11* | 26±9* |

| | | | |
|---|---|---|---|
| * p < 0.05 in comparison with the control group | | | |

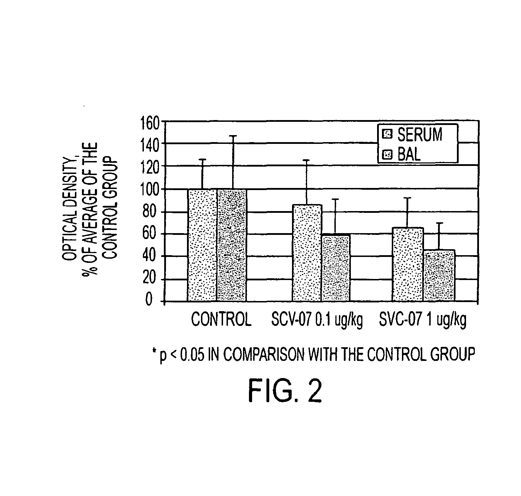

Measurement of the titers specific to the IgE antigen used in the model in the sera and bronchoalveolar lavage of the animals from 6 to 8 months of age [sic] show a significant reduction in titers in the serum of the group that receives 0.1 µg/kg SCV-07 in comparison with the control group. In the mice that received 1 µg/kg SCV-07, the titers of anti-OvA IgE were significantly lower both in the serum and in the bronchoalveolar liquid (see Table 3, Figure 2, wherein SCV-07 is referred to as SCV-07).

**Table 3. Titers of anti-OvA IgE (animals 6 to 8 weeks).**

| | Groups of animals | | |
|---|---|---|---|
| Index | Control | SCV-07 0.1 µg/kg | SCV-07 1 µg/kg |
| Titer IgE of serum, dilution 1:640, % of average of the control group | 100±26 | 86±42 | 65±26* |
| Titer IgE BAL, dilution 1:8, % of average of the control group | 100±47 | 59±33* | 46±24* |

| | | | |
|---|---|---|---|
| * p < 0.05 in comparison with the control group | | | |

In the experiments in which mice from 18 to 20 weeks of age were used, we also observed a tendency toward a reduction in the titers specific to IgE after using SCV-07, but in all groups the index had very high variability and the differences were not statistically significant (see Table 4).

**Table 4. Titers of anti-OvA IgE (animals 18 to 20 weeks).**

| | Groups of animals | | |
|---|---|---|---|
| Index | Control | SCV-07 0.1 µg/kg | SCV-07 1 µg/kg |
| Titer IgE of serum, dilution 1:400, % of average of the control group | 100±44 | 92±44 | 79±46 |
| Titer IgE BAL, dilution 1:32, % of average of the control group | 100±91 | 85±70 | 101±51 |

Morphological analysis of sections of the mice showed a more than two-fold reduction in the percentage of goblet cells in the epithelium of the bronchi of the animals that received 0.1 µg/kg SCV-07, significant in comparison with the control group (see Table 5). Morphometric evaluation of the intensity of peribronchial and perivascular infiltration has not been completed at present.

**Table 5. Percentage of goblet cells in the epithelium of the bronchi (animals 6 to 8 weeks).**

| | Groups of animals | | |
|---|---|---|---|
| Index | Control | SCV-07 0.1 µg/kg | SCV-07 1 µg/kg |
| % goblet cells of total number of cells | 39.3 | 15.2* | 36.5 |
| (median, minimum-maximum) | (3-85) | (4-90) | (3-92) |

| | | | |
|---|---|---|---|
| * p < 0.05 in comparison with the control group | | | |

Morphometric evaluation of the intensity of peribronchial and perivascular infiltration allowed a reliable decrease in the infiltration around the bronchi of both groups receiving SCV-07 to be demonstrated. The intensity of infiltration around the vessels also had a distinct tendency to decrease in the groups receiving SCV-07, however the differences from the control were not statistically reliable due to the high variability of symptoms (see Table 6).

**Table 6. Intensity of infiltration of the lung tissue.**

| | Groups of animals | | |
|---|---|---|---|
| Index | Control | SCV-07 0.1 µg/kg | SCV-07 1 µg/kg |
| Index of peribronchial infiltration, conventional units | 0.52±0.23 | 0.39±0.19* | 0.35±0.14** |
| Index of perivascular infiltration, conventional units | 2.46±2.22 | 1.67±1.08 | 1.48±1.02 |

| | | | |
|---|---|---|---|
| * p < 0.05 in comparison with the control group ** p < 0.01 in comparison with the control group | | | |

### CONCLUSION

The obtained results indicate that during use of SCV-07 in a model of allergic asthma a reduction in the severity of the experimental process is observed, specifically the emergence of leukocytes into the lumen of the bronchi and the peribronchial infiltration diminishes, eosinophilia of the bronchoalveolar lavage diminishes, the amount of antigen-specific IgE in the blood serum and lavage declines and the number of mucus-forming goblet cells in the epithelium of the bronchi also diminishes.

### Example 2. Effect of SCV-07 on production of cytokines by clones Th1 and Th2.

SCV-07 was administered to intact wild mice in the form of five daily intra-abdominal injections at dosages of 0.1 and 1.0 µg/kg of body weight. After this, isolation of spleen cells was carried out the cytokine synthesis was induced in the culture by stimulating the cells with concanavalin A at a dosage of 5 µg/mL. The levels of the synthesized cytokines were determined by quantitative immunoenzyme analysis. The results of the experiment are shown in Fig. 1, which shows the mitogen-induced production of interleukin-4 and interferon-γ by mouse splenocytes after intra-abdominal administration of SCV-07. The changes on ConA-induced production of two other [sic] cytokines - IFN-γ and IL-4, as is apparent from Fig. 1, were reciprocal in nature. Under the influence of SCV-07 (1 µg/kg), a two-fold reduction in production of IL-4 by the splenocytes and an almost five-fold increase in IFN-γ production were noted. These data suggests that SCV-07 not only stimulates the functional response of T-cells of the spleen, intensifying IL-2 production, but, at the same time, affects polarization of T-helpers by mostly activating the type 1 T-helper cells.

### Example 3. Treatment of bronchial asthma with SCV-07.

Patient B-skii, born 1952, reported with complaints of dyspnea attacks 6-7 times a day, cough with phlegm that was difficult to discharge, shortness of breath, and nasal obstruction. It is known from the history that, since 1985, he has suffered bronchial asthma, has been hospitalized several times by emergency medical services in the Allergological Department in conjunction with exacerbation of the disease. Since 1997, he has been taking systemic glucocorticoids (Prednisolone 2 tablets/day - maintenance dose). He is also receiving Beklazom LD 250 (up to 1000 µg/day), Flixotide 250 (to 1000 µg/day), prolonged broncholytics (Serevent 25 µg/unit 50 µg/s), Teotard 200 (2 k/day, 400 mg/day). The attacks are treated with salbutamol (Ventolin).

In 1994, he underwent allergology examination at the Municipal Allergy Office and household allergens were found (household dust, household dust mite); a dust ASIT was not carried out, in view of the serious course of the disease. The last exacerbation was 2 weeks ago, when attacks of dyspnea occurred on a background of the base therapy and were treated with difficulty with beta-2 agonists (short-term), and bronchospasm and shortness of breath increased. He was hospitalized at the Allergological Department of GUZ Hospital No. 2 in conjunction with refractoriness to outpatient treatment.

Heredity for allergic pathology was traced along the material line.
Allergy history:
- Drug - negative;
- Food - honey, citrus, watermelon, melon (nasal congestion, dyspnea attacks, cough);
- Domestic (household dust) - attacks of dyspnea, cough, nasal congestion, rhinorrhea;
- Epidermal - negative;
- Dust-seasonal exacerbation of rhinitis, conjunctivitis, increased frequency of dyspnea in the summer-fall period (July-October) for 10 years;
- Insect - negative;

On examination: state of average severity. Position of the patient induced (sitting). Skin cover- clean, pale, dry. Subcutaneous lymph nodes are not enlarged. Thyroid gland is not palpated. Nasal respiration is seriously hampered, sparse mucus is separated from the nasal passages; does not perceive odors. Conjunctivae - pink. Nasal mucosa (during rhinoscopy) - pale, edematous. Oral mucosa - pink. Dyspnea of expiratory character. Respiratory rate = 24 per minute. Chest cavity barrel-shaped. Percussion - capsular resonance of the pulmonary sounds. Auscultation - abundant dry rales, disseminated through all lung fields. Heart tones are rhythmic. HR = P5=78 per minute. **BP** = 130/85 mmHg.

Tongue is coated with white incrustation. Abdomen is soft, unpainful. Liver is along the edge of the costal arch. Region of the kidneys is not altered, Pasternak syndrome is negative on both sides.

On admission, an examination was conducted:
General blood analysis: erythrocytes - 5.07 ˙10¹²/L, hemoglobin = 167 g/L, leukocytes = 11.6 10⁹/L, ESR = 5 mm/h,
B = 1%, E = 3%, rod nuclears = 10%, segmented nuclears = 70%, lymphocytes = 11%, MON = 5%.

General sputum analysis: color - gray, character - mucous, constitution - viscous, flat epithelium - 10-12 x, alveolar epithelium - 3-5 x, L - 10-12 x, erythrocytes - none, goblet cells - not found.

Blood biochemistry: glucose - 5.9 mmol/L, total bilirubin - 11 µmol/L, ALT - 0.24, AST - 0.16 mmol/h/L, urea - 5.12 mmol/L, creatinine - 0.108 mmol/L, L-amylase - 7.0 mmol/L.
Hormonal status: serum cortisol - 39.8 nmol/L

Evaluation of immune status:

| | | |
|---|---|---|
| CD3⁺-53% | IgG-8.74 | TSIK- 40 relative units |
| CD4⁺-34% | IgA-0.67 | NST- 94/158/1.7 |
| CD8⁺-15% | IgM-1.39 | |
| CD16⁺-12% | IgE-1130.6 | |
| CD20⁺-18% | | |
| CD25⁺-1.3% | | |

| | | |
|---|---|---|
| EKG: Sinus rhythm. HR = 76 beats/minute Vision: without pathology FVD: FVC = 66%, FEV = 52%, FEV/FVC = 61 %, MOC75 = 37%, MOC50 = 34%, MOC25 = 51 %. PTM: L/exp - 2.0 Us L/exp - 1.0 Us. | | |

Chest x-ray: lung field emphysematous, diffuse pneumosclerosis in lower compartments. ENT consultation: Diagnosis: persistent allergic rhinitis. Chronic pharyngitis.

The patient was diagnosed as follows: DS: bronchial asthma, persistent, severe course, hormone-dependent, exacerbation. Complication: DN2. Emphysema of the lungs, pneumosclerosis.

Accompanying disease: persistent allergic rhinitis. Pollinosis, not exacerbated.
The patient was prescribed the treatment:
1. Ventolin through a nebulizer during dyspnea attacks - for 2 days, then Astalin (DAI) during dyspnea no more than 3-4 times a day;
2. Intravenous drip:
   1. Prednisolone 90 nu - 60 nu - 30 nu in 200.0 mL physiological saline;
   2. Euphylline 2.4%-10.0-5.0 mL.
   3. Heparin 5000 units.
   4. Benacort 200 (800 µg/day).
   5. Prednisolone 4 t/day (8:00, 12:00)
   6. Lazolvan through a nebulizer, then Ambrohexal 1/3 times a day.

During attenuation of the acute condition (on the 8^{th} day of hospitalization), SCV-07 was prescribed in an amount of 0.1 mg in 1.0 mL physiological saline once a day No. 5 daily [sic] intramuscular. After the course of treatment was performed (5 intramuscular injections), the patient was noted significant improvement of feeling well-being, positive mood, no side effects were noted, the preparation was well tolerated, body temperature was within normal values.

The patient took another examination under dynamic conditions (24 hours after the 5^{th} intramuscular injection of SCV-07):
Total blood analysis: erythrocytes - 5.12 10¹²/L, H_{B} - 159 g/L, leukocytes - 9.7 10⁹/L, ESR - 6 mm/h, b-, e - 2%, rod nuclears - 5%, segmented nuclears - 67%, lymphocytes - 20%, M - 6%.
Blood biochemistry: Sugar - 5.7; Total bilirubin - 8.9, ALT - 0.12, AST - 0.13, urea - 5.12, creatinine - 0.073
General sputum analysis - character - mucous, color gray, consistency viscous, flat epithelium 7-8, alveolar epithelium - none, leukocytes - 1-2 x in visual field.
Hormonal status - cortisol - 72.4

### Evaluation of immune status

| | | |
|---|---|---|
| CD4⁺- 36 | Ig G - 11.5 | |
| CD3⁺- 52 | Ig A - 1.11 | |
| CD8⁺- 15 | Ig M - 0.9 | |
| CD16⁺- 7 | Ig E - 672.1 | NST - 100/163 |
| CD20⁺-11 | | κ - 1.6 |
| CD25⁺-1.3 | TSIK - 77 | |

Positive dynamics of the disease was noted in the patient: elimination of dyspnea attacks, cough, rhinitis, reduction of shortness of breath (to 16 per minute), nasal congestion, patient began to sense odors.

The patient was released on the fifteenth day from the hospital in a satisfactory condition with corticosteroid therapy reduced to the maintenance dose (Prednisolone 2 tablets per day), and with a recommendation of base therapy and re-examination in 1 month.

### Example 4. Use of SCV-07 to reinforce the effectiveness of specific immune therapy of pollinosis.

Patient. - P-na M. N., 45 years. Date of first consultation - October 2004.
Diagnosis: Pollinosis with laboratory confirmed sensitization to tree pollen, rhinoconjunctival form, remission off-season.
The results of primary laboratory examination: moderate eosinophilia (7%), moderate hyper IgE immunoglobulinemia (165 ME/mL), high (MAST-class 3) levels of serum allergen-specific IgE to birch and hazelnut pollen, reduced (0.88) immune regulatory index.

From the end of November 2004 to mid-March 2005, the patient underwent specific immunotherapy (SIT) for pollinosis, using the allergoid ("Puretal-tree") according to the scheme: subcutaneous once a week, beginning with a single dose of the administered allergoid of 0.025 mL, the subsequent dose are escalated during the course of treatment until reaching a single dose of 0.5 mL (0.025 mL - 0.05 mL, - 0.1 mL - 0.2 mL - 0.3 mL - 0.4 mL - 0.5 mL), and the following does are staying on this dose.

The nature of the subsequent period of seasonal exacerbation of polinosis (April - June 2005) indicated extremely low effectiveness of the SIT: pronounced rhinoconjunctival symptoms still required the use of essential dose regimes of systemic peroral antihistamines (Erius) and topical steroid treatment (Nasonex).

Considering the above situation, during October 2005, to increase the clinical effectiveness of SIT, under initiating or anticipatory conditions, immune stimulation - a course of the preparation "SCV-07" -was perorally administered as follows: 1.5 mg once a day daily for 10 days in a row.

On completion of the "SCV-07" course, the patient, from the middle of November 2005 through mid-March 2006, underwent another course of SIT for pollinosis, using the allergoid "Puretal-tree" according to the previous scheme. The interval between the "SCV-07" course and the beginning of the SIT course for pollinosis was 15 days.

The nature of the subsequent period of seasonal exacerbation of pollinosis (April - June 2006) suggested a significantly increased the effectiveness of the performed SIT: seasonal rhinoconjunctival symptoms were virtually absent; neither antihistamine preparations nor topical steroids were used in the patient during the season of tree pollination.

The obtained results indicate the effectiveness of using "SCV-07" to prevent and treat allergic diseases. The harmlessness of the preparation and its relatively low cost make it promising for widespread use for this purpose as an independent therapy and in the context of integrated therapy.

### Example 5: EFFECTIVENESS OF SCV-07 IN A GUINEA PIG MODEL OF ALLERGIC DISEASE

### Summary

Asthma is lung disorder characterized by bronchoconstriction, inflammatory cell infiltration into the lung (particularly eosinophils), airway hyperresponsiveness and increased mucus secretion. Allergic asthma can be modeled in guinea pigs with a significant immediate bronchoconstrictor response to allergen as well as an inflammatory cell infiltration and lung injury that is apparent 24 hours after allergen exposure. The purpose of the present study was to determine if SCV-07 could attenuate inflammatory cell infiltration into the lung after allergen challenge. Guinea pigs were sensitized by ip injection of ovalbumin. On days 17-21, they were treated ip with PBS vehicle, SCV-07 at 1 ug/kg or SCV-07 at 10 ug/kg. On day 21, all animals were pre-treated with antihistamine to reduce the immediate bronchoconstrictor response that can be fatal. Subsequently, animals were exposed to aerosolized ovalbumin to initiate the allergic reaction. Control animals were sensitized with ovalbumin and challenged with saline aerosol as control.

### SCV-07 treatment

- Reduced cough, labored breathing, and general distress of the animals during the immediate allergic reaction.
- Reduced the resident eosinophils and neutrophils in the lung tissue
- Reduced allergic lung damage as evidenced by a significant attenuation of red blood cell leakage in the bronchoalveolar lavage
- Did not appreciably inhibit inflammatory cell infiltration into the allergic lung or the ovalbumin-specific antibody production

Conclusion: SCV-07 shows promise in reducing the immediate allergic reaction and subsequent lung damage, but is ineffective in preventing inflammatory cell infiltration into the allergic lung. Future studies should examine the ability of SCV-07 to inhibit allergen-induced bronchoconstriction. In addition, given the protective effect of SCV-07 on red blood cell leakage, its ability to prevent allergen-induced changes in microvascular permeability as well as mast cell mediator release should be assessed.

### Abbreviations

- EPO: eosinophil peroxidase
- MPO: myeloperoxidase
- WBC: white blood cell
- RBC: red blood cell
- BAL: bronchoalveolar lavage
- NSS: normal saline solution
- PBS: phosphate buffered saline
- OVA: ovalbumin
- ip: intraperitoneal

Background: Anecdotal evidence suggests that humans with chronic asthma receiving SCV-07 for other indications were able to reduce their use of rescue medications for their asthma. Assumption: The use of □₂ agonists in patients was reduced suggesting that airway hyperresponsiveness and/or allergen induced early and late phase bronchoconstriction was attenuated by SCV-07. Studies in a mouse model of asthma also suggest that cellular infiltration may be slightly reduced after allergen challenge (SciClone communication).

Purpose: To determine if SCV-07 alleviates asthma symptoms in a guinea pig model of asthma.

Specific Aim: Determine if administration of SCV-07 after sensitization but prior to allergen challenge reduces cellular infiltration in a guinea pig model of asthma.

### Materials and Methods

### Animals

Female Dunkin-Hartley guinea pigs (200-350 g) were purchased from Charles River, Kingston, New York facility, Barrier K81. Animals were shipped to Duluth, Minnesota, and held in our animal facility for 5-7 days prior to beginning the experiment. Experimental treatment groups are outlined in Table 7.

**Table 7: Experimental Treatment Groups**

| Name of Treatment Group | Sensitization | Peptide treatment | Aerosol challenge | N |
|---|---|---|---|---|
| PBS NSS | OVA | PBS | NSS | 8 |
| PBS OVA | OVA | PBS | OVA | 7 |
| SCV 1 NSS | OVA | SCV-07 1 g/kg | NSS | 8 |
| SCV 1 OVA | OVA | SCV-07 1 g/kg | OVA | 8 |
| SCV 10 NSS | OVA | SCV-07 10 ug/kg | NSS | 8 |
| SCV 10 OVA | OVA | SCV-07 10 ug/kg | OVA | 7 |

Five separate experiments with 8-10 animals per experiment were conducted over an approximately 6 month period. The first experiment consisted of 4 PBS NSS animals and 4 PBS OVA animals to insure that the experimental system was working properly and that the OVA challenged animals developed significant eosinophilia. The subsequent 4 experiments contained anywhere from 1-3 animals from each of the 6 treatment groups for a final N of 7-8 animals per treatment group as shown in Table 7.

### Experimental Procedure

Animals were sensitized on day 0 ip with 50 mg/kg OVA. On Days 17, 18, 19, and 20, animals received an ip injection between 8 and 10 am with PBS, SCV-07 at 1 ug/kg, or SCV-07 at 10 ug/kg (Peptide treatment). Between 8 and 10 a.m. on Day 21, animals were administered peptide 2 hours before OVA or saline (NSS) aerosol challenge. 30 min before OVA or NSS aerosol challenge each animal was injected ip with an antihistamine (6.1 mg/kg pyrilamine maleate) to prevent death due to the anaphylactic response that accompanies aerosol OVA challenge. Animals were exposed to aerosol in pairs with either 1% OVA solution or NSS for 5 min in a plexiglass chamber (22 X 22 X 29 cm) using a DeVilbiss Model 35B Ultrasonic Nebulizer. Animals were observed during the total 5 min aerosolization and comments recorded. Within 22-24 hours after challenge, animals were euthanized, bled by cardiac puncture, lavaged and lung lobes removed for measurement of cellular infiltration. At the time of OVA or NSS challenge and euthanasia, animal weights ranged from approximately 350-550 g, with the average weight approximately 420 g. This experimental protocol is similar to that used in previously published studies (Regal and Fraser, 1996; Regal et al., 2000).

### Determination of cellular infiltration

Animals were euthanized with pentobarbital (100-200 mg/kg), bled by cardiac puncture, and lavaged with room temperature PBS. For bronchoalveolar lavage (BAL), the trachea was cannulated and 4 volumes of PBS were introduced into the tracheal cannula and gently withdrawn. Total lavage volume was 60 ml/kg. The BAL was centrifuged to sediment the cells, and the BAL cell pellet re-suspended in 1.0 ml PBS for determination of the total number of white blood cells (WBC) recovered in the BAL of each animal. The BAL supernatant was frozen at -70 C for determination of total protein recovered in the BAL supernatant using the method of Lowry et al. (1951). Total white blood cells in BAL were counted by standard methods in a hemacytometer using Turk's solution. Differential counts were obtained from cytospin preparations of BAL cells (3 X 10⁴ cells) stained with a modified Wrights' stain (Diff Quik, American Scientific Products, McGraw Park, IL). Four hundred cells were counted and identified as eosinophils, neutrophils, or mononuclear cells. The BAL cell differential and white blood cell counts were used to calculate the total number of each cell type recovered per animal from the BAL. For estimates of the numbers of eosinophils and neutrophils in the lung tissue, the left lung lobe was processed for measurement of eosinophil peroxidase (EPO) and the right lung lobe for myeloperoxidase (MPO) activity, respectively (Fraser et al., 1995). The remainder of the lung was dried at 80° C for 3-5 days for determination of gram dry weight. EPO activity from the homogenized lung after lavage was expressed as total OD/min from the left caudal lobe. MPO activity from the homogenized lung after lavage was expressed as total units of enzyme activity from the right caudal lobe. SCV-07 at 1 ug/ml did not inhibit the EPO or MPO assay. The number of RBC in the BAL was_quantified by determining the OD₄₁₂ of the re-suspended BAL cell pellet after lysis of the RBC as previously described (Fraser et al., 1995). OD₄₁₂ of the BAL supernatant was also determined to estimate the number of RBCs that were lysed during the lavage procedure and prior to re-suspension of the cells for counting. SCV-07 at 0.3 ug/ml did not interfere with OD 412 measurements in vitro.

### OVA specific IgG1

OVA specific IgG1 in serum was determined by ELISA as previously described (Fraser et al., 1998; Regal et al., 2000). The data were expressed as the concentration of OVA specific IgG1 in the sample divided by the concentration of OVA specific IgG1 in the standard, defined as 1. The IgG standard was prepared by passing a pool of serum from OVA sensitized guinea pigs over a Protein A Sepharose column. The recovered IgG was dialyzed against NSS and aliquoted for use as a standard in the assay.

### Reagents

### SCV-07

200 mg SCV-07 was shipped to Duluth by SciClone and stored at -70 C. The compound was weighed in the laboratory with lights out and stored in the dark. Stock solutions of 20 ug/ml and 2 ug/ml SCV-07 were made in PBS and stored in polypropylene tubes (Sarstedt tubes; Ref 72.694.105) in 1 ml aliquots in the -70 C freezer. The PBS used to make up the SCV-07 solutions was also aliquoted and frozen for control PBS injections (PBS Catalog number, Gibco 10010, no calcium or magnesium, Endotoxin tested as less than 0.03 EU/ml). Aliquots were thawed the day of use and kept on ice in the dark in foil wrapped tubes. If not completely used that day, they were stored in the dark in the refrigerator and used up the next day, or discarded. Animals were administered 0.5 ml/kg of PBS, 2 ug/ml SCV-07 or 20 ug/ml SCV-07 resulting in a delivered dose of SCV 1 (1 ug/kg) or SCV 10 (10ug/kg).

### OVA for sensitization

100 ml of a solution of Ovalbumin (A5503 from Sigma) at 25 mg/ml in saline (Baxter, Sterile, nonpyrogenic) was prepared. Four ml aliquots were frozen at -70 in Nunc cryotube vials (Cat 337516). Animals were administered 2 ml/kg of this OVA solution ip with a 21 g needle resulting in a final delivered dose of 50 mg/kg. All animals in this experiment were sensitized with OVA.

### OVA for aerosol challenge.

1 % OVA solution was prepared fresh the day of the aerosol challenge in room temperature NSS (Baxter, Sterile, nonpyrogenic).

### Statistical analysis

All data were log transformed to equalize the variances and allow normal parametric modeling. For values of OVA specific IgG1, prior to statistical analysis a constant of 0.01 was added to all values to minimize the impact of very low values on variances in the log scale. Values in all figures represent the geometric mean + standard error (SE) of 7-8 values. Statistical analysis used ANOVA with contrasts using JMP software (SAS Institute Inc., Cary, N.C., USA). Statistical significance was defined as p<0.05.

### Results

### Effect of SCV-07 on the immediate response to OVA aerosol

Animals were observed during OVA aerosolization, and the comments recorded are summarized in Table 8. The experimenter was not blinded to the treatment. As seen in the left side of Table 8, animals challenged with NSS aerosol exhibited normal behavior and no changes in breathing pattern or movement were recorded. In general, animals sensitized and challenged with OVA and antihistamine pre-treated will not die from the allergen challenge, but will experience labored breathing and oftentimes coughing and gasping. All 7 animals in the PBS OVA treatment group had some visible reaction as expected. The remarkable finding was the number of animals in the SCV 1 OVA and SCV 10 OVA treatment groups that had no apparent reaction to the OVA aerosol. These data suggest that SCV-07 pretreatment at either 1 or 10 ug/kg is protective of the immediate anaphylactic effects of allergen sensitization and challenge. The obvious speculation is that SCV-07 attenuates the immediate bronchoconstriction to allergen challenge. Follow-up experiments are definitely indicated by this observation.

**Table 8 Comments recorded during aerosolization of guinea pigs (46 animals)**

| **Treatment** | **Comment** | **Treatment** | **Comment** |
|---|---|---|---|
| PBS NSS | huddling in corner, no rx | PBS OVA | slight cough |
| PBS NSS | huddling in corner, no rx | PBS OVA | slight cough |
| PBS NSS | huddling in corner, no rx | PBS OVA | panting after removed from chamber |
| PBS NSS | huddling in corner, no rx | PBS OVA | flopping upside down, very distressed at 4 min |
| PBS NSS | huddling in corner, no rx | PBS OVA | flopping upside down, very distressed at ∼2 min |
| PBS NSS | no rx | PBS OVA | coughing ∼3min distressed |
| PBS NSS | no rx | PBS OVA | panting, distressed |
| PBS NSS | no rx | | |
| SCV 1 NSS | huddling in corner | SCV 1 OVA | no rx |
| SCV 1 NSS | huddling in corner | SCV 1 OVA | slight cough 1 x ? |
| SCV 1 NSS | huddling in corner | SCV 1 OVA | no rx |
| SCV 1 NSS | no rx | SCV 1 OVA | no rx |
| SCV 1 NSS | no rx | SCV 1 OVA | no rx |
| SCV 1 NSS | no rx | SCV 1 OVA | 1 cough ? |
| SCV 1 NSS | no rx | SCV 1 OVA | no rx |
| SCV 1 NSS | no rx | SCV 1 OVA | coughing near end of aerosolization |
| SCV 10 NSS | huddling in corner, no rx | SCV 10 OVA | no rx |
| SCV 10 | huddling in corner, no rx | SCV 10 | slight cough |
| NSS | | OVA | |
| SCV 10 NSS | no rx | SCV 10 OVA | no rx |
| SCV 10 NSS | no rx | SCV 10 OVA | no rx |
| SCV 10 NSS | no rx | SCV 10 OVA | 1 cough ? |
| SCV 10 NSS | no rx | SCV 10 OVA | deeper breathing |
| SCV 10 NSS | no rx | SCV 10 OVA | deeper breathing |
| SCV 10 NSS | no rx | | |

### Effect of SCV-07 on OVA-induced cellular infiltration into the lung

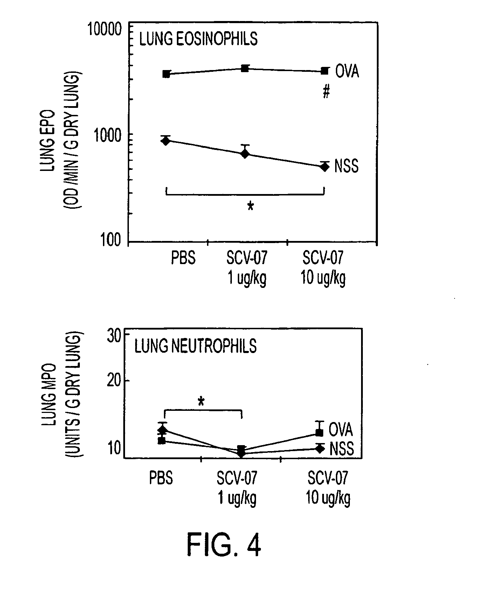

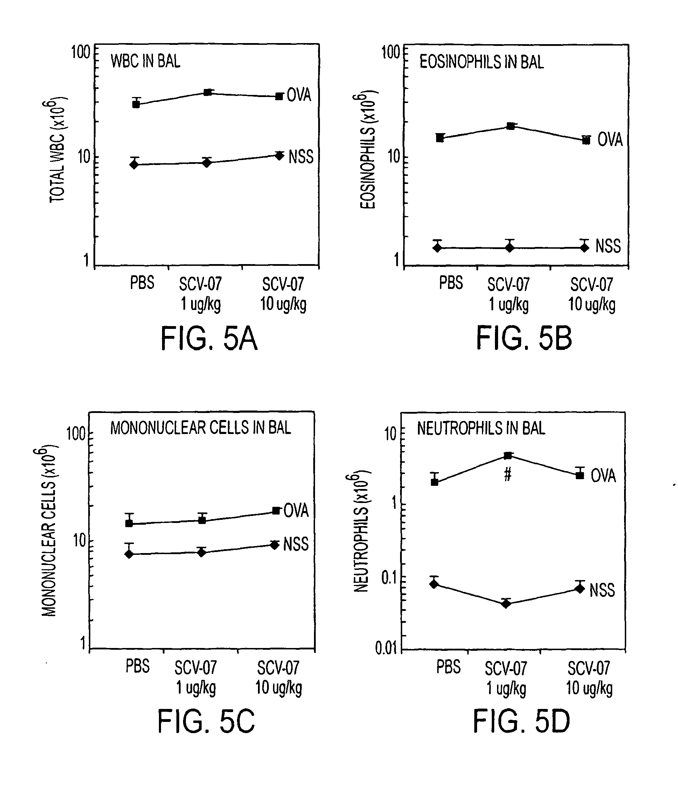

All animals were sensitized with OVA and the effect of SCV-07 was determined both on animals challenged with NSS aerosol (negative control, no allergic reaction) and animals challenged with OVA aerosol. The eosinophils in the lung tissue were estimated by measurement of eosinophil peroxidase in homogenized lung, and the neutrophils in the lung tissue estimated by measurement of myeloperoxidase (Fig 4). Previous studies, both from our lab and others, have validated these measurements as estimates of inflammatory cells in tissue. In addition, we determined the numbers and types of white blood cell that were present in the airspace, i.e. BAL cells (Fig 5-6).

### Effect of SCV-07 on resident cells in the lung

Statistical analysis initially involved ANOVA with contrasts of control animals challenged with NSS aerosol only (n=24) to determine if there was a significant effect of SCV-07 on resident eosinophils or neutrophils in the lung tissue or airspace. As seen in Fig 4, SCV-07 at 1 ug/kg significantly reduced the MPO in animals challenged with NSS aerosol, and SCV-07 at 10 ug/kg significantly reduced the EPO (*p<0.05 vs PBS NSS control). These data suggest that in the lung tissue, SCV-07 treatment significantly reduces the number of resident eosinophils and neutrophils (Fig 4). However, in the BAL of control animals challenged with NSS, SCV-07 did not significantly affect the composition of cells (Fig 5, NSS).

### Effect of SCV-07 on OVA-induced cellular infiltration

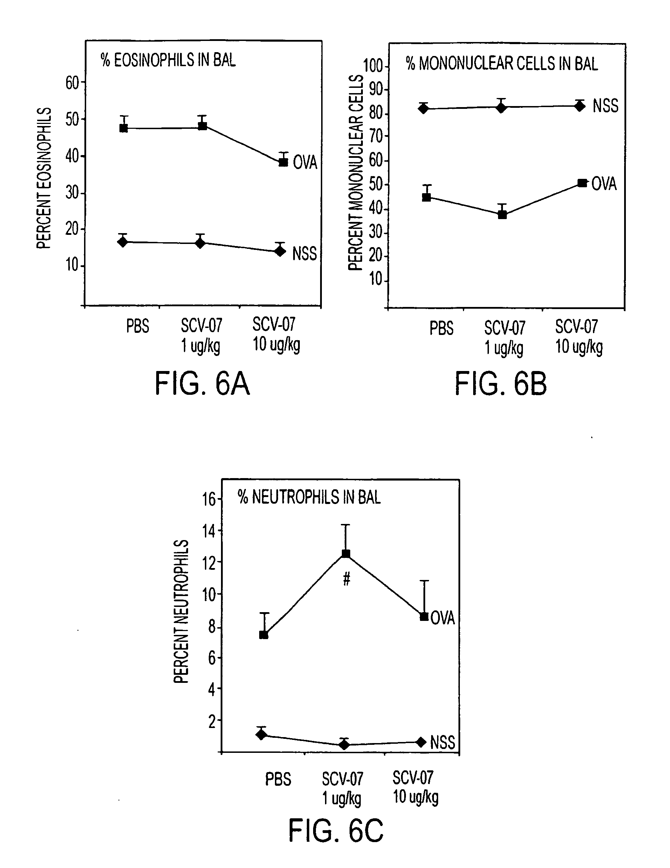

As evident in Figures 4 and 5, ANOVA analysis indicated a significant overall OVA effect in lung EPO, as well as all of the BAL cell types. Consistent with our previous results, lung MPO was not significantly increased with OVA challenge. Since the resident eosinophils and neutrophils changed with SCV-07 treatment, we did the following ANOVA contrasts:
(Change from PBS NSS to PBS OVA) vs (Change from SCV 1 NSS to SCV 1 OVA)
(Change from PBS NSS to PBS OVA) vs (Change from SCV 10 NSS to SCV 10 OVA)
As seen in Fig 4, the change in lung EPO was greater with 10 ug/kg SCV-07 treatment than with PBS treatment (#). The change in lung MPO did not differ with SCV-07 treatment. Considering the BAL cells, the change in BAL neutrophils differed with 1 ug/kg SCV-07 treatment. In part, these differences were due to the decrease in resident eosinophils and neutrophils with SCV-07 treatment. The cell differentials expressed as % are also presented in Figure 6 A-C for comparison to the study of SCV-07 in the mouse asthma model (SciClone doc 43-10718). The % eosinophils in the BAL tended to decrease in SCV-07 treated animals with OVA aerosol challenge (Fig 6A). However, the total eosinophils in the BAL did not change (Fig 5B).

### Effect of SCV-07 on OVA-induced lung injury and/or changes in microvascular permeability

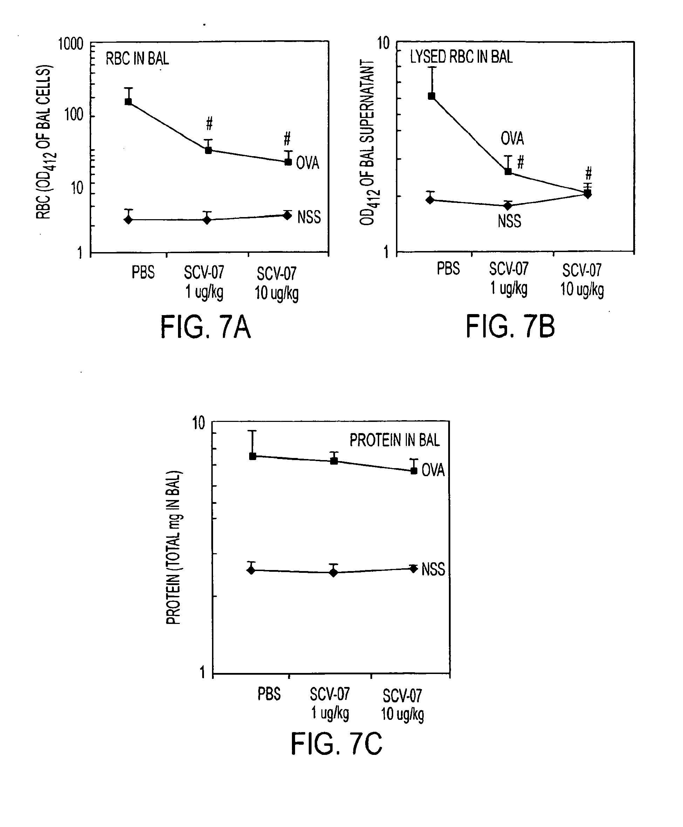

Allergen challenge is known to cause increased microvascular permeability in the lung as well as lung injury that can result in leakage of red blood cells into the airspace. Either of these events could result in increased protein content of the BAL fluid due to leakage of plasma proteins or interstitial fluid into the airspace. Lung injury and/or changes in microvascular permeability were assessed by determining the RBCs in the BAL (Fig 7A-B) as well as the overall protein content of the BAL fluid (Fig 7C). The number of RBCs in the BAL was estimated by lysing the cell pellet and measuring the OD₄₁₂ of the released hemoglobin (Fig 7A). In addition, OD₄₁₂ of the BAL supernatant was also determined to estimate the number of RBCs that were lysed prior to or during the lavage procedure, and prior to re-suspension of the cells for counting (Fig 7B). As evident in Figure 7, OVA challenge of a PBS treated animal significantly increased the number of RBCs in the BAL and the total protein in the BAL. ANOVA contrasts of the change from NSS to OVA indicated that SCV-07 at either 1 or 10 ug/kg significantly attenuated the number of RBCs in the BAL (#, Fig 7A). In addition, the OD₄₁₂ of the BAL supernatant was also significantly reduced by SCV-07 indicating that significantly fewer RBCs were available to be lysed in the process of collecting lavage fluid (#, Fig 7B). The total protein in the BAL fluid was not affected by SCV-07 treatment (Fig 7C).

### Effect of SCV-07 on serum OVA-specific IgG1 antibody

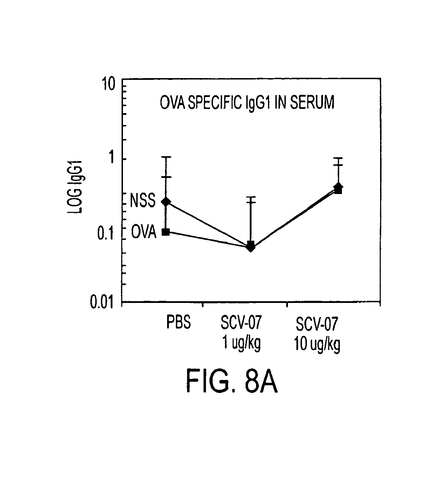

Mast cell sensitizing antibodies involved in allergic lung responses in the guinea pig include both IgE and IgG1 antibody. Guinea pigs sensitized with OVA in the absence of adjuvant primarily make OVA specific IgG1 antibody. Previous studies in our lab have determined that the method used to sensitize animals in this study is unlikely to result in production of OVA specific IgE (Fraser et al., 1995; Regal and Fraser, 1996). To insure that any effects seen with SCV-07 treatment were not due to alterations in OVA specific IgG1 antibody produced, the concentrations in serum were determined by ELISA (Fig. 8). Since all animals were sensitized with OVA in this study, OVA specific IgG1 antibody was detectable in all groups. SCV-07 treatment or OVA challenge did not significantly affect the concentration of OVA specific IgG1 antibody in the serum.

### General Conclusions and Discussion

### Asthma Control is a Significant Health Care Issue

Recent studies clearly suggest that the majority of asthmatics are not well controlled with available drugs. The Real world Evaluation of Asthma Control and Treatment (REACT) study indicates that uncontrolled asthma is highly prevalent. Their results suggested that 55% of patients using standard asthma medications had uncontrolled asthma. This occurred despite the vast majority of these patients having access to health care (Peters et al., 2007). It is unclear whether this lack of asthma control results from the sub-optimal use of existing medications, or from the fact that existing medications do not target the correct mediators and/or events in many asthmatics. Thus, new asthma therapies are needed, whether they target the immediate response to allergen or the more chronic inflammatory effects of allergen exposure. Certainly there is evidence that the release of mediators in the immediate allergic reaction puts events in motion that contribute to the more chronic effects of the inflammatory disease (Paul, 1999).

### Main findings of the present study in the guinea pig model Immediate allergic response

SCV-07 treatment in the guinea pig was observed to reduce cough, labored breathing, and general distress of the animals during the immediate allergic reaction seen with OVA aerosol challenge. The previous study of the mouse model did not report any findings regarding the immediate allergic response during aerosol allergen challenge. In general, mice respond to allergen challenge with minimal changes in pulmonary function compared to the guinea pig, and mice do not require antihistamine pre-treatment to prevent death during the immediate allergic reactions. Our observation of the SCV-07 effect on the immediate response in the guinea pig model suggests a very clinically useful property of SCV-07 in allergic disease. The utility of SCV-07 treatment in immediate allergic reactions in the skin and lung should be investigated further.

### Allergic lung inflammation

Guinea pigs respond to allergen sensitization and challenge with infiltration of inflammatory cells into the lung within 24 hours after allergen challenge. Five days of SCV-07 pre-treatment did not inhibit changes in eosinophil infiltration into the guinea pig lung when the total number of cells was examined (Fig 5). SCV-07 tended to decrease the % of eosinophils accumulated after allergen challenge in the BAL (Fig 6). However, this was not statistically significant. Whether the percentage of cell type in the airspace is of clinical importance or the total number of a cell type in the airspace is the important variable to evaluate can be debated. Evaluation of either of these (Fig 5-6) results in the same conclusion. SCV-07 treatment did not appreciably inhibit allergen induced inflammatory cell infiltration into the guinea pig lung. However, SCV-07 treatment clearly reduced the total lung EPO and MPO in control animals, suggesting that the compound was affecting the number of resident eosinophils and neutrophils in the lung in the normal state. This finding needs to be followed up by histopathological examination of lungs of guinea pigs in the different treatment groups to see if SCV-07 treatment alone alters the number of resident cells.
Previous research in model systems of allergy and asthma with SCV-07 are limited. I have reviewed the study of SCV-07 in a mouse model of asthma (SciClone Doc 43-10718). In the mouse study, two different age groups of mice were sensitized and challenged with OVA and cellular infiltration reported. The rationale for conducting the study in two different age groups of mice was not evident. In our study with guinea pigs, only one age group was evaluated. We chose to use female guinea pigs of 200-350 grams because our previous studies in young and adult, male and female guinea pigs indicated that animals of this sex and age consistently produced a strong allergic lung inflammation (Regal et al., 2006). These animals are actively growing over the course of the study, and animals of this weight range are commonly used in guinea pig models of allergic inflammation. In the mouse studies reported, a modest decrease in the % of eosinophils in the BAL of 6-8 wk mice was reported with no changes in total cell numbers. In 18-20 wk old animals, no differences were seen in percentages of cell types with SCV-07 treatment, but SCV-07 decreased the total eosinophils in the BAL of the 18-20 wk old. In the mouse study, cell differentials or total counts from BAL for unsensitized animals or animals sensitized but not challenged with aerosol OVA are not reported. A normal guinea pig has a resident population of eosinophils in the lung, whereas eosinophils are not as apparent in a normal mouse lung. In our study, because of the effect of SCV-07 treatment on the baseline EPO and MPO measurements, it was important to statistically analyze the change in the variable between NSS and OVA aerosol with the appropriate control peptide pre-treated group. No such comparison was available for the mouse data.

### Lung damage or changes in microvascular permeability

A severe allergic reaction in the guinea pig lung is accompanied by increased numbers of red blood cells in the BAL 24 hours later. Clearly SCV-07 significantly inhibited this event (Fig. 7). Whether this reflects the observed attenuation of the immediate allergic response in the animal (the first 5 min) or reflects damage that occurs in the lung after the immediate response but within the first 24 hours cannot be determined from our study design. However, the remarkable difference in the number of RBCs in the BAL after SCV-07 treatment suggests that the compound has a very significant protective effect. This protective effect has very important clinical implications and should be followed up with future experiments.

### Humoral immune response

SCV-07 treatment was begun 17 days after the initial sensitization with OVA so that the primary immune response was not likely to be affected by peptide treatment. Serum concentrations of OVA specific IgG1 were determined at the time of lavage after allergen challenge. As expected, there was no significant difference in serum levels of IgG1 in the treatment groups. This indicates that any changes seen in the allergic response are not likely due to differences in the availability of cytophilic antibody to mediate the response. This differs in part from the mouse study where a small but significant effect on OVA-specific IgE was noted in SCV-07 treated mice. However in the mouse model, SCV-07 treatment differed and began only one day after the second sensitization injection of OVA plus alum.

### Strengths and limitations of the study

Whether the guinea pig model of allergic lung inflammation reflects asthma in the human can always be debated. An advantage of the guinea pig model is that eosinophil infiltration into the lung readily occurs. Additionally, the guinea pig readily produces antibody to OVA in the absence of adjuvant. A disadvantage is that guinea pigs do not readily produce IgE antibody like humans but produce cytophilic IgG1 antibody. As in humans, the guinea pig has a marked immediate bronchoconstrictor response to allergen challenge whereas this response in the mouse model is minimal and short-lived. Also, the guinea pig must be pre-treated with an antihistamine or up to 50% of them will die during the 5 min aerosol in our model. Thus, this additional drug treatment may complicate the interpretation.

Measurement of EPO and MPO provides a picture of the total eosinophils and neutrophils in the lung lobe, respectively, but does not provide information as to the location of those cells. Follow-up histopathology is necessary to examine the location of the inflammatory cells and confirm the biochemical findings. Comments recorded during the immediate response suggest that SCV-07 protected animals from the immediate bronchoconstrictor response due to allergen exposure. However, measurements of pulmonary function need to follow up this intriguing observation.

### Conclusions

In this robust guinea pig model of asthma, SCV-07 shows promise in reducing the immediate allergic reaction and subsequent lung damage, but is ineffective in preventing inflammatory cell infiltration into the allergic lung. Future studies should examine the ability of SCV-07 to inhibit allergen-induced bronchoconstriction. In addition, given the protective effect of SCV-07 on lung damage, its ability to prevent allergen-induced changes in microvascular permeability as well as mast cell mediator release in skin and lung should be assessed.

### References

Fraser DG, Regal JF, and Amdt ML: Trimellitic anhydride-induced allergic response in the lung: Role of the complement system in cellular changes. J. Pharmacol exp Ther 273:793-801, 1995.
Fraser DG, Graziano FM, Larsen CP and Regal JF: The role of IgG1 and IgG2 in trimellitic anhydride-induced allergic response in the guinea pig lung. Toxicology and Applied Pharmacology 150: 218-227, 1998.
Larsen CP and Regal JF: Trimellitic anhydride-induced cellular infiltration into guinea pig lung varies with age but not gender. Int Arch Allergy Immunol 127:63-72, 2002.
Lowry OJ, Rosebrough NJ, Farr AL, Randall PJ: Protein measurement with the Folin phenol reagent. J Biol Chem 1951;193:265-275.
Paul WE: Fundamental Immunology, 4th edition, 1999. Lippincott-Raven Publishers, Philadelphia.
Peters SP, Jones CA, Haselkorn T, Mink DR, Valacer DJ, and Weiss ST: Real-world evaluation of asthma control and treatment (REACT): Finding from a national Web-based survey. J Allergy Clin Immunol 119: 1454-1461, 2007.
Regal JF and Fraser DG: Systemic complement system depletion does not inhibit cellular accumulation in antihistamine pretreated allergic guinea pig lung. Int Arch Allergy Immunol 109:150-160, 1996.
Regal JF, Fraser DG, Weeks CD, Greenberg NA: Dietary phytoestrogens have antiinflammatory activity in a guinea pig model of asthma. PSEBM 223:372-378, 2000.
Regal JF, Regal RR, Meehan JL, Mohrman ME: Primary prevention of asthma: Age and sex influence sensitivity to allergen-induced airway inflammation and contribute to asthma heterogeneity in guinea pigs. Int Arch Allergy Immunol 141:241-256, 2006.

## Claims

1. An immunomodulator compound of Formula A, or a pharmaceutically acceptable salt thereof, for use in treating, preventing, inhibiting or reducing allergic disease or its effects in a subject, 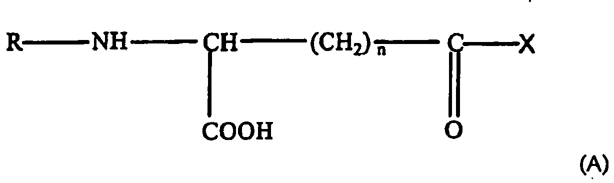 wherein, n is for use according to 2; R is hydrogen, acyl, alkyl or a peptide fragment; and X is L-typtophan or D-typtophan.

2. The compound for use according to claim 1, wherein said compound is γ-D-glutamyl-L-tryptophan.

3. The compound for use according to claim 1 or 2, wherein said disease is asthma.

4. The compound for use according to claim 3, wherein said asthma is atopic bronchial asthma.

5. The compound for use according to any one of claims 1 to 4, wherein said compound is administered at a dosage within a range of about 0.001-10 mg.

6. The compound for use according to any one of claims 1 to 4, wherein said compound is administered at a dosage within a range of about 0.01-1 mg.

7. The compound for use according to any one of claims 1 to 4, wherein said compound is administered at a dosage within a range of about 0.0001-100 mg/kg subject body weight.

8. The compound for use according to any one of claims 1 to 4, wherein said compound is administered at a dosage within a range of about 0.001-1 mg/kg subject body weight.

9. The compound for use according to any one of claims 1 to 8, wherein said γ-D-glutamyl-L-tryptophan is in an isotonic solution for parenteral administration at a daily dosage of about 0.01 to 1 mg.

10. The compound for use according to any one of claims 1 to 8, wherein said γ-D-glutamyl-L-tryptophan is in a preparation for peroral administration at a daily dosage up to about 1.5 mg.

11. The compound for use according to any one of claims 1 to 8, wherein γ-D-glutamyt-L-tryptophan is in a preparation for administration once a day with a course of from about 5 to 14 days.

12. Use of an immunomodulator compound as claimed in anyone of claims 1 to 11 for the preparation of a medicament for treating, preventing, inhibiting or reducing allergic disease or its effects in a subject.

## Patentansprüche

1. Immunomodulator-Verbindung der Formel A oder ein pharmazeutisch annehmbares Salz davon zur Verwendung beim Behandeln, Verhindern, Hemmen oder Verringern einer allergischen Erkrankung oder ihrer Wirkungen bei einem Individuum, 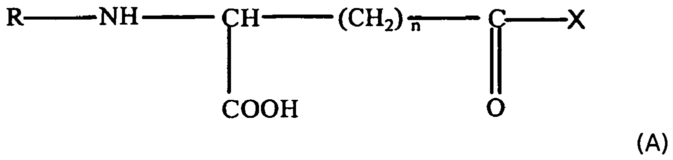 worin n 2 ist; R Wasserstoff, Acyl, Alkyl oder ein Peptid-Fragment ist; und X L-Tryptophan oder D-Tryptophan ist.

2. Verbindung zur Verwendung nach Anspruch 1, wobei die Verbindung γ-D-Glutamyl-L-tryptophan ist.

3. Verbindung zur Verwendung nach Anspruch 1 oder 2, wobei die Erkrankung Asthma ist.

4. Verbindung zur Verwendung nach Anspruch 3, wobei das Asthma atopisches Bronchialasthma ist.

5. Verbindung zur Verwendung nach einem der Ansprüche 1 bis 4, wobei die Verbindung in einer Dosierung in einem Bereich von etwa 0,001 bis 10 mg verabreicht wird.

6. Verbindung zur Verwendung nach einem der Ansprüche 1 bis 4, wobei die Verbindung in einer Dosierung in einem Bereich von etwa 0,01 bis 1 mg verabreicht wird.

7. Verbindung zur Verwendung nach einem der Ansprüche 1 bis 4, wobei die Verbindung in einer Dosierung in einem Bereich von etwa 0,0001 bis 100 mg/kg Körpergewicht des Individuums verabreicht wird.

8. Verbindung zur Verwendung nach einem der Ansprüche 1 bis 4, wobei die Verbindung in eine Dosierung in einem Bereich von etwa 0,001 bis 1 mg/kg Körpergewicht des Individuums verabreicht wird.

9. Verbindung zur Verwendung nach einem der Ansprüche 1 bis 8, wobei das γ-D-Glutamyl-L-tryptophan sich in einer isotonischen Lösung zur parenteralen Verabreichung mit einer täglichen Dosierung von etwa 0,01 bis 1 mg befindet.

10. Verbindung zur Verwendung nach einem der Ansprüche 1 bis 8, wobei das γ-D-Glutamyl-L-tryptophan sich in einem Präparat zur peroralen Verabreichung mit einer täglichen Dosierung von bis zu etwa 1,5 mg befindet.

11. Verbindung zur Verwendung nach einem der Ansprüche 1 bis 8, wobei sich γ-D-Glutamyl-L-tryptophan in einem Präparat zur Verabreichung einmal täglich mit einer Behandlungsdauer von etwa 5 bis 14 Tagen befindet.

12. Verwendung einer Immunomodulator-Verbindung wie in einem der Ansprüche 1 bis 11 beansprucht zur Herstellung eines Arzneimittels zum Behandeln, Verhindern, Hemmen oder Verringern allergischer Erkrankungen oder ihrer Wirkungen bei einem Individuum.

## Revendications

1. Composé immunomodulateur de formule A, ou un sel acceptable sur le plan pharmaceutique de celui-ci, destiné à une utilisation dans le traitement, la prévention, l'inhibition ou la réduction d'une maladie allergique ou des effets de celle-ci sur un sujet, 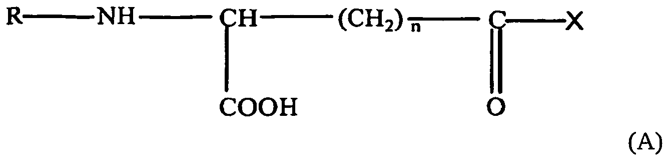 dans lequel n est 2 ; R est de l'hydrogène, un acyle, un alkyle ou un fragment peptidique, et X est du L-tryptophane ou du D-tryptophane.

2. Composé destiné à une utilisation selon la revendication 1, dans lequel le dit composé est du γ-D-glutamyl-L-tryptophane.

3. Composé destiné à une utilisation selon la revendication 1 ou 2, dans lequel ladite maladie est l'asthme.

4. Composé destiné à une utilisation selon la revendication 3, dans lequel ledit asthme est de l'asthme bronchique atopique.

5. Composé destiné à une utilisation selon l'une quelconque des revendications 1 à 4, dans lequel ledit composé est administré avec un dosage compris dans les limites d'un intervalle d'environ 0,001-10 mg.

6. Composé destiné à une utilisation selon l'une quelconque des revendications 1 à 4, dans lequel ledit composé est administré avec un dosage compris dans les limites d'un intervalle d'environ 0,01-1 mg.

7. Composé destiné à une utilisation selon l'une quelconque des revendications 1 à 4, dans lequel ledit composé est administré avec un dosage compris dans les limites d'un intervalle d'environ 0,0001-100 mg/kg du poids corporel du sujet.

8. Composé destiné à une utilisation selon l'une quelconque des revendications 1 à 4, dans lequel ledit composé est administré avec un dosage compris dans les limites d'un intervalle d'environ 0,001-1 mg/kg du poids corporel du sujet.

9. Composé destiné à une utilisation selon l'une quelconque des revendications 1 à 8, dans lequel ledit γ-D-glutamyl-L-tryptophane est une solution isotonique destinée à une administration parentérale avec un dosage quotidien d'environ 0,01 à 1 mg.

10. Composé destiné à une utilisation selon l'une quelconque des revendications 1 à 8, dans lequel ledit γ-D-glutamyl-L-tryptophane se trouve dans une préparation destinée à une administration perorale avec un dosage quotidien allant jusqu'à 1,5 mg.

11. Composé destiné à une utilisation selon l'une quelconque des revendications 1 à 8, dans lequel ledit γ-D-glutamyl-L-tryptophane se trouve dans une préparation destinée à une administration d'une fois par jour sur une période allant d'environ 5 à 14 jours.

12. Utilisation d'un composé immunomodulateur selon l'une quelconque des revendications 1 à 11, pour la préparation d'un médicament destiné au traitement, la prévention, l'inhibition ou la réduction d'une maladie allergique ou des effets de celle-ci sur un sujet.
